# EUROPEAN PATENT SPECIFICATION

(11) **EP 3 181 545 B1**
(45) Date of publication and mention of the grant of the patent: **01.11.2023**
(21) Application number: 15842755.9
(22) Date of filing: 15.09.2015
(51) Int. Cl.: C07C 67/14, C07C 67/29, C07C 67/31, C07C 69/78, C07C 69/736, C07C 37/20, C07C 39/373, C07C 41/26, C07C 43/23

(54) **PROCESS FOR THE PREPARATION OF OSPEMIFENE**
VERFAHREN ZUR HERSTELLUNG VON OSPEMIFEN
PROCÉDÉ DE PRÉPARATION D'OSPÉMIFÈNE

(30) Priority: 16.09.2014 JP 2014187228
(43) Date of publication of application: 21.06.2017
(73) Proprietor: Shionogi & Co., Ltd., Osaka-shi, Osaka 541-0045 (JP)
(72) Inventor: OKAMOTO, Kazuya, Toyonaka-shi Osaka 561-0825 (JP); UENO, Tatsuhiko, Toyonaka-shi Osaka 561-0825 (JP); TOYOTA, Takashi, Isawa-gun, Iwate 0294503 (JP); SATOU, Yuusuke, Amagasaki-shi Hyogo 660-0813 (JP)
(74) Representative: Eder, Michael
(86) International application number: PCT/JP2015/076165
(87) International publication number: WO 2016/043189

(56) References cited:
- WO-A1-01/36360
- WO-A1-86/05777
- WO-A1-2008/099059
- WO-A1-2009/081789
- WO-A1-2011/089385
- WO-A1-2014/060640
- WO-A1-2014/060640
- CN-A- 104 030 896
- JP-A- H05 271 130
- JP-A- H05 271 130
- JP-A- H07 509 222
- JP-A- H10 502 924
- JP-A- H11 310 556
- JP-A- H11 310 556
- JP-A- 2007 517 796
- JP-A- 2010 518 150
- US-A1- 2012 053 341
- LUKÁC, I. ET AL.: "Synthesis of acyl 1-acetoxy-2-phenoxyethanes and the corresponding hydroxy derivatives", COLLECTION CZECHOSLOV. CHEM. COMMUN., vol. 45, no. 6, 1980, pages 1826-1830, XP55457280, ISSN: 0010-0765, DOI: 10.1135/cccc19801826 ISBN: 978-80-86241-25-8
- SUAREZ, A.G.: "AlCl3-DMA reagent in the regioselective solvent free Friedel-Crafts acylation reaction of benzodioxin derivatives", TETRAHEDRON LETTERS, vol. 40, no. 18, 30 April 1999 (1999-04-30), pages 3523-3526, XP004162326, ISSN: 0040-4039, DOI: 10.1016/S0040-4039(99)00514-6
- RELE, S.M. ET AL.: "Salt/ligand-activated low-valent titanium formulations: the 'salt effect' on diastereoselective carbon-carbon bond forming SET reactions", TETRAHEDRON, vol. 64, no. 30-31, 2008, pages 7225-7233, XP022762796, ISSN: 0040-4020, DOI: 10.1016/J.TET.2008.05.084 [retrieved on 2008-05-21]
- ZHANG, D. ET AL.: "Two-step synthesis technology of ospemifene", XIANDAI YAOWU YU LINCHUANG - DRUGS & CLINIC, TAINJIN ZHONGCAOYAO ZAZHISHE, CN, vol. 27, no. 4, 29 March 2012 (2012-03-29) , pages 351-352, XP002719433, ISSN: 1674-5515

## Description

### [Technical Field]

This invention relates to a process for the preparation of triphenylbutene derivatives, more particularly to a process for the preparation of Ospemifene, which is a selective estrogen modulator.

### [Background Art]

Patent Documents 1 to 5 disclose a compound of the formula (VII): 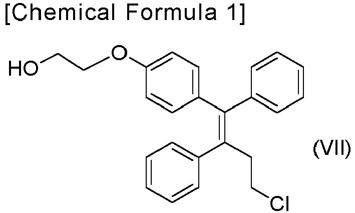 which is a selective estrogen receptor modulators.

Patent Document 1 describes the process for the preparation of Ospemifene, by reacting 4-hydroxybenzophenone with 3-chloropropiophenone by McMurry reaction to obtain a compound represented by the Formula (II): 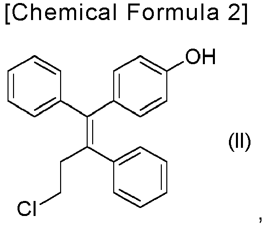 followed by alkylation with alkylation agent represented by the Formula: X-(CH₂)₂-O-Pr, wherein X is Cl, Br, I, mesyloxy or tosyloxy, Pr is a protecting group, to obtain a compound represented by the Formula (IV): 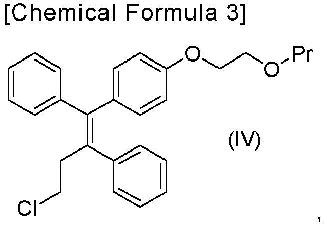 followed by deprotection to obtain Ospemifene, and by alkylation with alkylation agent represented by the Formula: X-(CH₂)₂-O-COOR, wherein X is Cl, Br, I, mesyloxy or tosyloxy, R is alkyl, to obtain a compound represented by the Formula (V): 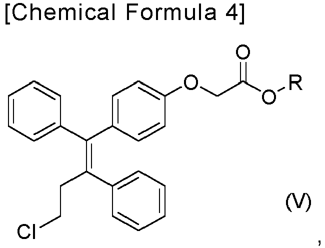 followed by reducing the ester to obtain Ospemifene.

Patent Document 2 describes the process for the preparation of Ospemifene, by reacting a compound represented by the Formula (II): 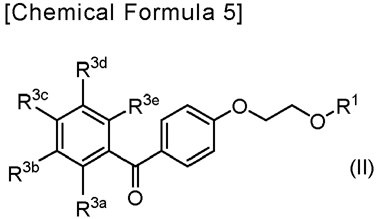 wherein R¹ is C1 -C6 alkyl optionally substituted with one or more -OH group, each R^{3a}, R^{3b}, R^{3c}, R^{3d} and R^{3e} is independently H or -OH,
with a compound represented by the Formula (III): 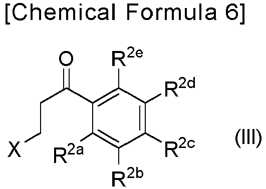 wherein X is halo or -OH, each R^{2a}, R^{2b}, R^{2c}, R^{2d} and R^{2e} is independently H or -OF by McMurry reaction.

Patent Document 3 describes the process for the preparation of Ospemifene, by reacting a compound represented by the Formula (Illa): 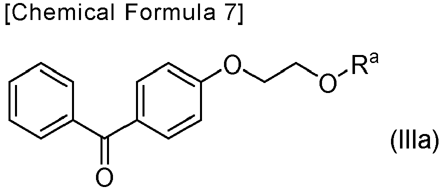 wherein R^{a} is C(O)-R^{b}, R^{b} is optionally substituted phenyl,
with 3-chloro-propiophenone by McMurry reaction to obtain a compound represented by the Formula (lVa): 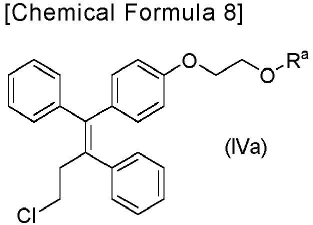 wherein the symbol in the Formula has the same meaning as defined above, followed by deprotecting.

Patent Document 4 describes the process for the preparation of Ospemifene, by reacting a compound represented by the Formula (III): 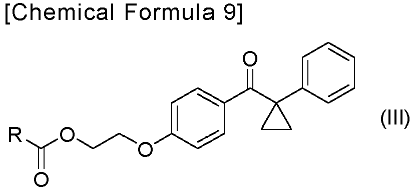 with phenyl magnesium halide to obtain a compound represented by the Formula (IV): 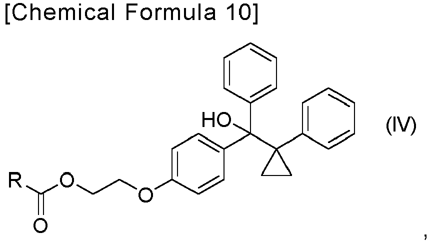 followed by treatment with hydrochloric acid to obtain a compound represented by the Formula (V): 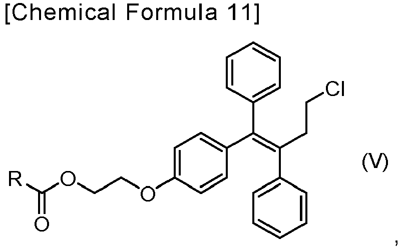 followed by deprotecting.

Patent Document 5 describes the process for the preparation of Ospemifene characterized by introducing a perfluorophenyl group.

The Scheme 1 in Non-Patent Document 1 describes the process for the preparation of 2- (4-acetylphenyl) ethyl acetate by reacting 2-phenylethanol with acetyl chloride in the presence of aluminum chloride. However the reaction yield is 50% and low.

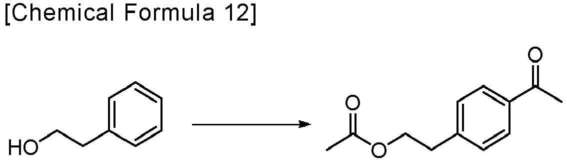

Non-Patent Document 2 describes the addition of an alcohol in a Pinacol reaction before a McMurry reaction can improve diastereoselectivity.

Non- Patent Document 3 describes the addition of alkali metal chloride to a titanium reagent can improve the activity of the titanium reagent.

However, Patent Documents 1 to 5 or non- Patent Documents 1 to 3 do not describe or suggest the process for the preparation of Ospemifene using a McMurry coupling reaction in the presence of an alkali metal salt and/or substituted or unsubstituted phenol, or using reducing reaction with sodium borohydride.

### [Prior Art Documents]

### Patent Documents

[Patent Document 1] WO2008/099059
[Patent Document 2] WO2011/089385
[Patent Document 3] WO2014/060640
[Patent Document 4] WO2014/060639
[Patent Document 5] Chinese Application Publication CN10324214

### Non-Patent Documents

[Non-Patent Document 1] Synthesis (1993) 1261-1265
[Non-Patent Document 2] Journal of the American Chemical Society 1996, 118, 5932-5937
[Non-Patent Document 3] Tetrahedron 64 (2008) 7225-7233

### [Disclosure of Invention]

### [Problems to be solved by the Invention]

The purpose of the present invention is to provide a novel and useful process for preparing triphenyl-butene derivative represented by the Formula (VII), also known as Ospemifene.

### [Means for Solving the Problem]

Example 1 in Patent Document 1 describes the process for the preparation of 4-(4-chloro-1,2-diphenyl - but-1-enyl) phenol by McMurry reaction of 4-hydroxybenzophenone with 3-chloro-propiophenone But the yield of the process is low despite the lack of a purification step. Moreover it is difficult to obtain a product with high purity.

Additionally, as the esters obtained by the method described in Example 6 are oily, intermediates, their use in the commercial process not preferred. Further the process for the preparation described in Example 7 describes the process for the preparation of Ospemifene, by reducing [4-(4-chloro-1,2-diphenyl-but-1-enyl)- phenoxy - acetic acid ethyl ester with lithium aluminum hydride. The yield of process for the preparation is low. Moreover more explosive lithium aluminum hydride is used.

Example 1A and 1B in Patent Document 2 describe the process for the preparation of Ospemifene by McMurry reaction of 4-(2-hydroxyethoxy) benzophenone with 3-chloro-propiophenone. But the yield of the process is low despite the lack of a purification step. Moreover its yield is worse than that of the present invention.

Example 8 and 9 in Patent Document 3 describe the process for the preparation of 2- (4-benzoyl-phenoxy) ethyl benzoate. Its yield is worse than that of the present invention.

Example 10 describes the process for the preparation of (Z-)-2-(4- (4-chloro-1,2 diphenyl-but-1-yl) phenoxy) ethyl benzoate by McMurry reaction of 2-(4-benzoylphenoxy) ethyl benzoate with 3-chloropropiophenone. Its yield is worse than that of the present invention.

Also, Example 11 describes the process for the preparation of Ospemifene, by reducing (Z) -2- (4- (4- chloro-1,2-diphenyl - but-1-yl) phenoxy) ethyl benzoate with lithium aluminum hydride. But the yield of process for the preparation is low. Moreover explosive lithium aluminum hydride is used.

The present inventors have found a process for the preparation of triphenylbutene derivatives of the formula (VII) and its intermediate. In contrast to the previous processes, the disclosed process has a good yield without the use of an explosive reagent. Therefore, COGS (cost of goods sold) of the present invention is excellent, making the present invention suitable for industrial use. Further, a compound of the formula (X') is a useful compound as an intermediate. A compound of the formula (VII) can be prepared via the intermediate effectively.

That said, the present invention specifically relates to a process for the preparation of a compound represented by formula (VII): 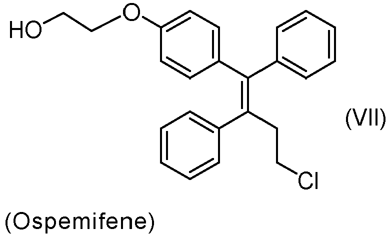 characterized by reacting a compound represented by Formula (X'): 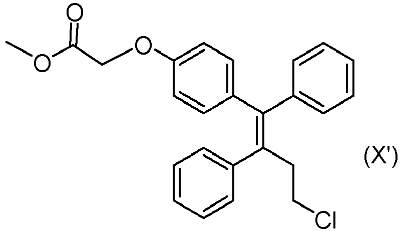 with sodium borohydride.

In some embodiments, the amount of the sodium borohydride is from 1 mol to 5 mol equivalents to the compound of Formula (X').

In some embodiments, the solvent is one or more solvents selected from the group consisting of methanol, ethanol, isopropanol, n-propanol, tert-butanol, n-butanol, 1,2-dimethoxyethane, N, N-dimethylformamide, N,N-dimethylacetoamide, N-methyl pyrrolidone, 1,3-dimethyl-2-imidazolidinone, toluene, cyclopentylmethylether, tetrahydrofuran, 2-methyl tetrahydrofuran, and dimethylsulfoxide.

In some embodiments the solvent is preferably a polar solvent. For example, in certain embodiments, the solvent is one or more solvents selected from the group consisting of methanol, ethanol, isopropanol, n-propanol, tert-butanol, n-butanol, 1,2-dimethoxyethane, N, N-dimethylformamide, N,N-dimethylacetoamide, N-methyl pyrrolidone, 1,3-dimethyl-2-imidazolidinone, cyclopentylmethylether, tetrahydrofuran, 2-methyl tetrahydrofuran, and dimethylsulfoxide. In other embodiments, the solvent is preferably a mixture of tetrahydrofuran and methanol.

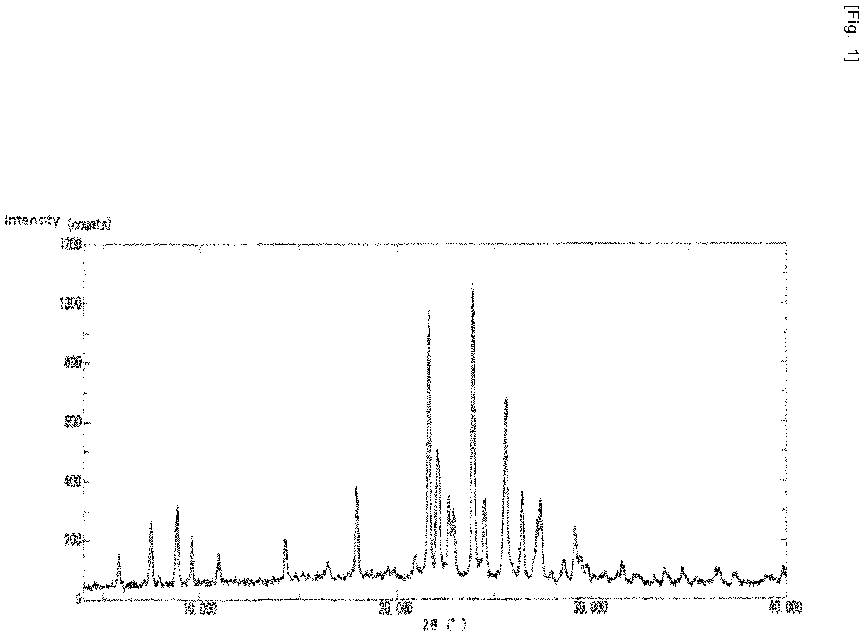

The present disclosure furthermore describes the following items (not according to the invention as claimed):
(1) A process for the preparation of a compound represented by Formula (I): 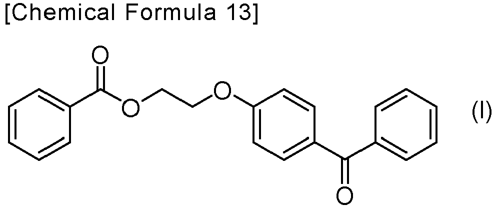 characterized by reacting a compound represented by Formula (II): 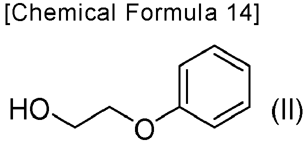 with a compound represented by Formula (III): 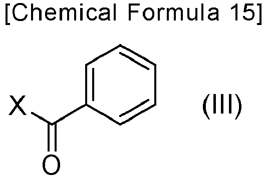 wherein X is halogen,
   in the presence of a Lewis acid.
(2) The process for the preparation of the above (1), wherein the Lewis acid is an aluminum chloride, titanium(IV) chloride, tin(IV) chloride, or boron trifluoride.
(3) The process for the preparation of the above (1), wherein the Lewis acid is an aluminum chloride.
(4) The process for the preparation according to any one of the above (1) to (3), wherein X is chloride.
(5) A process for the preparation of a compound represented by Formula (IV): 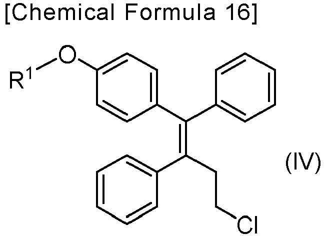 wherein R¹ is hydrogen or a substituted or unsubstituted alkyl,
   characterized by reacting a compound represented by Formula (V): 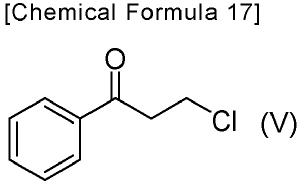 with a compound represented by Formula (VI): 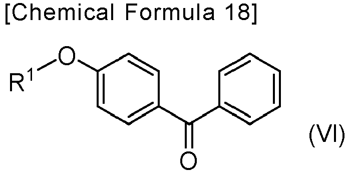 wherein R¹ has the same meaning as defined above,
   in the presence of
   1) a polyvalent metal chloride,
   2) a reducing agent and
   3) an alkali metal salt and/or a substituted or unsubstituted phenol.
(6) The process for the preparation of the above (5), wherein the alkali metal salt is potassium chloride.
(7) The process for the preparation of the above (5) or (6), wherein the substituent of the substituted phenol is one or more substituents selected from the group consisting of fluoride, chloride, bromide, cyano, trifluoromethyl, hydroxy and nitro.
(8) The process for the preparation of the above (5) or (6), characterized by reacting in the presence of orthochlorophenol.
(9) The process according to any one of the above (5) to (8), wherein the polyvalent metal chloride is titanium chloride compound.
(10) The process for the preparation of the above (9), wherein the polyvalent metal chloride is titanium(IV) chloride.
(11) The process according to any one of the above (5) to (10), wherein the reducing agent is zinc, copper, lithium, magnesium, aluminum, lithium aluminum hydride or trimethylamine.
(12) The process for the preparation of the above (11), wherein the reducing agent is zinc.
(13) The process according to any one of (5) to (12), characterized by reacting in the solvent comprising 2-methyltetrahydrofuran.
(14) The process according to any one of the above (5) to (12), characterized by reacting in the mixed solvent comprising 2-methyltetrahydrofuran and toluene.
(15) The process according to any one of the above (5) to (14), wherein R¹ is hydrogen.
(16) The process according to any one of the above (5) to (14), wherein R¹ is a substituted alkyl, and said substituted alkyl is a group represented by Formula: - CH₂CH₂OR², wherein R² is hydrogen or a protecting group.
(17) The process for the preparation of the above (16), wherein R² is benzoyl.
(18) The process according to any one of the above (5) to (14), (16) and (17), which comprises preparing the compound represented by Formula (VI-1): 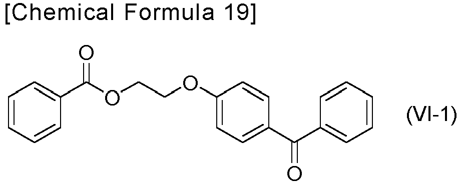 by the process described in any one of the above (1) to (4).
(19) A process for the purification of crude product containing a compound represented by Formula (IV): 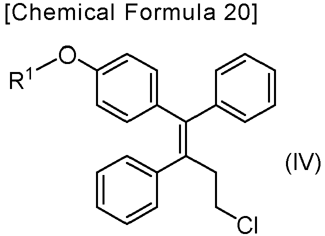 wherein R¹ has the same meaning as defined at the above (5),
   characterized by purifying said compound with the mixed solvent comprising methanol/water = 4/2 - 4/1.
(20) A process for the preparation of a compound represented by Formula (IV): 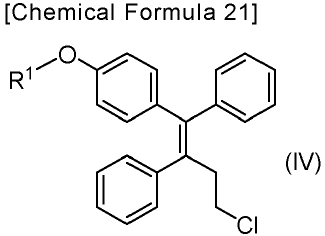 wherein R¹ is hydrogen or a substituted or unsubstituted alkyl,
   characterized by reacting a compound represented by Formula (V): 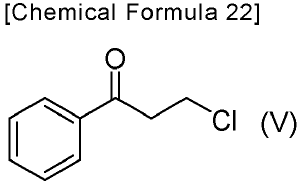 with a compound represented by Formula (VI): 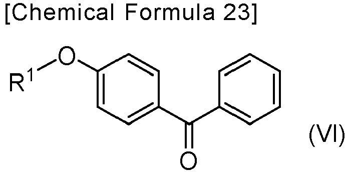 wherein R¹ has the same meaning as defined above,
   in the presence of 1) a polyvalent metal chloride, and 2) a reducing agent, then purifying the obtained compound with mixed solvent comprising methanol/water = 4/2 - 4/1.
(21) A process for the preparation of a compound represented by Formula (VII): 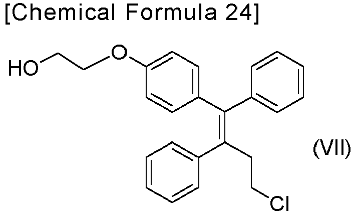 characterized by reacting a compound represented by Formula (X): 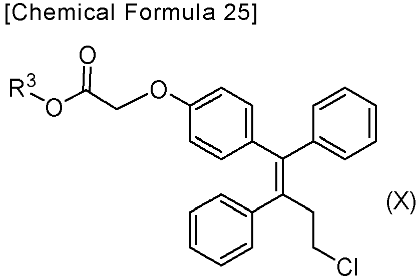 wherein R³ is a substituted or unsubstituted alkyl or a substituted or unsubstituted aromatic carbocyclyl,
   with a sodium borohydride.
(22) The process for the preparation of the above (21), wherein R³ is a substituted or unsubstituted alkyl.
(23) The process for the preparation of a compound represented by Formula (VII): 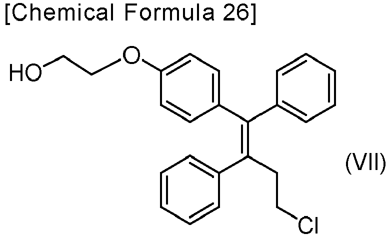 which comprises a process according to any one of the above items (5) to (18).
(24) A compound represented by Formula (X'): 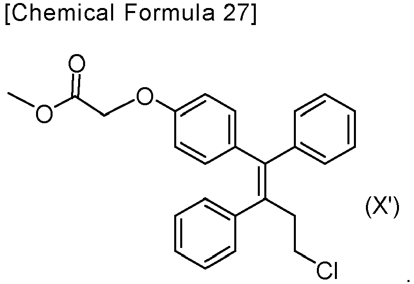
(25) A crystal of the compound represented by Formula (X'): 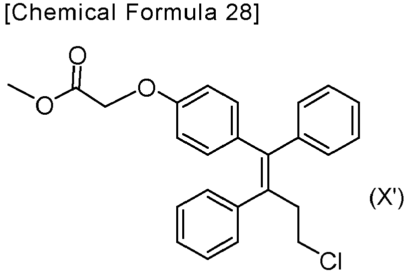 in the above (24).
(26) The crystal of a compound of the above (25), wherein the values of 2θ of the powder X-ray diffraction has 2θ of 18.0°±0.2°, 21.6°±0.2°, 22.1°±0.2°, 23.9°±0.2° and 25.6°±0.2° degrees.
(27) The crystal of a compound of the above (25), wherein the values of 2θ of the powder X-ray diffraction has 2θ of 8.8°±0.2°, 18.0°±0.2°, 21.6°±0.2°, 22.1°±0.2°, 23.9°±0.2°, 25.6°±0.2°and 26.4±0.2° degrees.
(28) The crystal of a compound of item 25, characterized by a powder X-ray diffraction spectrum substantially consistent in FIG. 1.

### [Effect of the Invention]

A process for the preparation of the present invention can be used to prepare Ospemifene effectively.

### [Mode for Carrying Out the Invention]

In the following, meanings of terms used in the present specification will be explained.

"Halogen" includes fluorine, chlorine, bromine or iodine. Fluorine and chlorine are preferred.

"Alkyl" means a straight or branched hydrocarbon group having 1 to 6 carbon atoms, and includes alkyl of 1 to 4 carbon atoms, alkyl of 1 to 3 carbon atoms and the like. Examples include methyl, ethyl, n-propyl, isopropyl, n-butyl, isobutyl, sec-butyl, tert-butyl, n-pentyl, isopentyl, neopentyl, hexyl, isohexyl and the like.

Preferable examples of "alkyl" for R¹ include ethyl.

"Aromatic carbocyclyl" includes monocyclic or polycyclic aromatic carbocyclic groups and groups wherein such monocyclic or polycyclic aromatic carbocyclic ring is fused with further one or two 3- to 8-membered rings. Specific examples of the monocyclic or polycyclic aromatic carbocyclic group include phenyl, naphthyl, anthryl and phenanthryl. Particularly, phenyl is preferred.

Specific examples of the ring to be fused with the monocyclic or polycyclic aromatic carbocyclic group include non-aromatic carbocycles such as cycloalkane rings (for example: cyclohexane ring, cyclopentane ring etc.), cycloalkene rings (for example: cyclohexene ring, cyclopentene ring etc.), and non-aromatic heterocycles (for example: piperidine ring, piperazine ring, morpholine ring etc). The monocyclic or polycyclic aromatic carbocyclyl should be involved in the linkage of such fused ring.

Examples of the aromatic carbocyclic groups include the following groups.

These groups may have a substituent at any possible position.

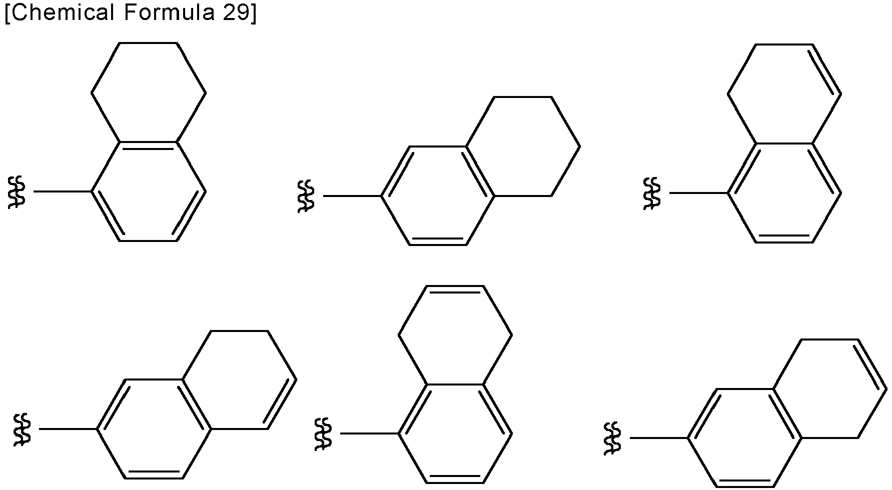

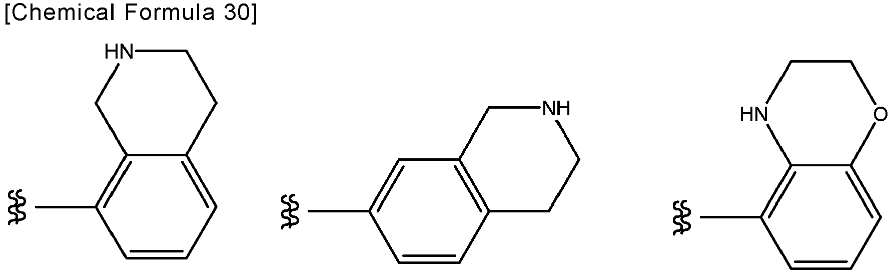

Examples of the substituent group for "substituted alkyl" or "substituted aromatic carbocyclyl" include halogen, hydroxy, mercapto, nitro, nitroso, cyano, azido, formyl, amino, carboxy, alkyl, haloalkyl, alkenyl, alkynyl, non-aromatic carbocyclyl, aromatic carbocyclyl, aromatic heterocyclyl, non-aromatic heterocyclyl, substituted carbamoyl, substituted sulfamoyl, substituted amidino, a group of formula: -O-R^{x}, a group of formula: -O-C(=O)-R^{x}, a group of formula: -C(=O)-R^{x}, a group of formula : - C(=O)-O-R^{x}, a group of formula: -S-R^{x} or a group formula: -SO₂ -R^{x} wherein R^{x} is alkyl, haloalkyl, alkenyl, alkynyl, non-aromatic carbocyclyl, aromatic carbocyclyl, aromatic heterocyclyl, non-aromatic heterocyclyl, carbamoyl, sulfamoyl or amidino. One or more of these substituents may occur at any substitutable position.

Examples of the substituent for "substituted alkyl" in R¹ include for example, hydroxy, alkyloxy, (hydroxyalkyloxy, phenylalkyloxy, etc.), non-aromatic carbocyclyloxy (tetrahydropyranyloxy, etc.), alkylcarbonyloxy (methylcarbonyloxy, ethylcarbonyloxy, etc.), aromatic carbocyclylcarbonyloxy (phenylcarbonyloxy, etc.), acyl (acetyl, trichloroacetyl, benzoyl, etc.), alkyloxycarbonyl (t-butoxycarbonyl, etc.), alkylsulfonyl (methanesulfonyl, etc. ), alkyloxyalkyl (methoxymethyl, etc.), trialkylsilyl (t-butyldimethylsilyl, etc.) and the like. Hydroxy, alkyloxy, non-aromatic carbocyclyloxy, alkylcarbonyloxy, aromatic carbocyclylcarbonyloxy and the like are preferable.

Examples of salt include salts with inorganic acids such as hydrochloric acid, sulfuric acid, nitric acid, phosphoric acid and the like; and organic acids such as acetic acid, formic acid, p-toluenesulfonic acid, methanesulfonic acid, oxalic acid, citric acid and the like.

Examples of solvates include a hydrate, an alcohol solvate and the like including a compound or its salt. Examples of a solvate are 1 hydrate, 2 hydrates, 1 alcohol solvate, 2 alcohols, a solvate of a compound or its salt.

"Polyvalent metal chloride" means a compound in which a metal ion having an ionic valence of 2 or more is ionically bonded with a chloride ion. Titanium chloride (e.g., titanium tetrachloride, titanium trichloride, etc.), aluminum chloride and the like is preferable. Titanium tetrachloride is especially preferable.

Examples of "protecting group" for R² include the protecting group hydroxy. A substituted or unsubstituted alkylcarbonyl or a substituted or unsubstituted aromatic carbocyclylcarbonyl is preferred. Additionally alkylcarbonyl or phenylcarbonyl are preferable, methylcarbony is especially preferable

Reaction of a compound with a compound includes reaction of the salt of each compound or solvate thereof in the present description.

The process disclosed herein (not according to the invention as claimed) can be conducted as follows:

### Step 1

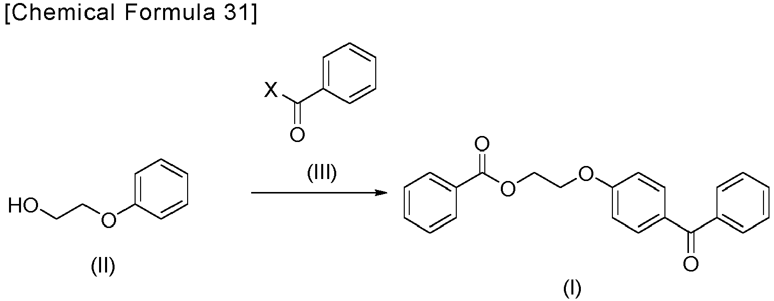

Wherein X is halogen.

Compound (I) can be obtained by reacting Compound (II) with Compound (III) in the presence of a Lewis acid in solvent.

A Lewis acid is not limited as long as it efficiently proceeds in the above process. An aluminum chloride, titanium(IV) chloride, tin(IV) chloride, or boron trifluoride can be used. Preferable examples include an aluminum chloride.

The amount of the Lewis acid can be 2 mol to 5 mol equivalents, preferably 2 mol to 3 mol equivalents to Compound (II).

The amount of Compound (III) can be 1 mol to 5 mol equivalent(s), preferably 2 mol to 3 mol equivalents to Compound (II).

A solvent is not limited as long as it efficiently proceeds in the above process. Examples of a solvent include dichloromethane, toluene, tetrahydrofuran and the like. Preferable solvent includes dichloromethane.

The temperature for such reaction is not limited, but usually can be about 0 to 100 °C and preferably at room temperature.

Reaction time is not limited, but usually can be conducted for 0.5 to 20 hours and preferably 1 to 5 hour(s).

### Step 2

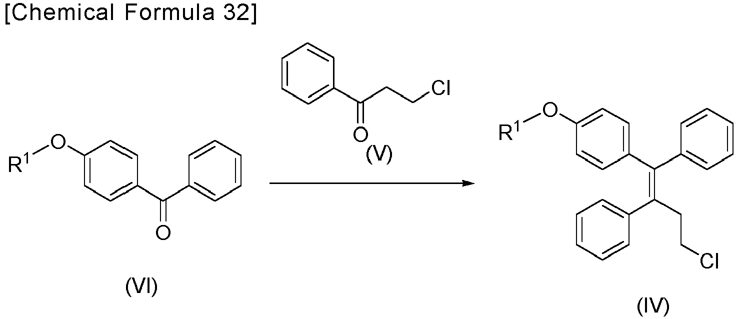

Wherein R¹ is hydrogen or a substituted or unsubstituted alkyl.

Compound (IV) can be obtained by reacting Compound (VI) with Compound (V) in the presence of 1) a polyvalent metal chloride, 2) a reducing agent and 3) an alkali metal salt and/or a substituted or unsubstituted phenol in a solvent.

A polyvalent metal chloride is not limited as long as it efficiently proceeds in the above process. Preferable examples of the polyvalent metal chloride include titanium chloride (for example, titanium(IV) chloride, titanium(III) chloride and the like), aluminium chloride and the like. Especially preferable examples include titanium(IV) chloride.

The amount of the polyvalent metal chloride may be 2 to 10 mole equivalents, preferably 2 to 5 mole equivalents of Compound (VI).

Examples of the reducing agent include zinc, copper, lithium, magnesium, aluminum, lithium hydride, aluminum, iron, magnesium, triethylamine and the like. Especially preferable examples include zinc.

The amount of the reducing agent may be 1 to 10 mole equivalents, preferably 3 to 5 mole equivalents of Compound (VI).

Examples of the alkali metal salt include fluorinated alkyl metals (e.g., lithium fluoride, sodium fluoride, potassium fluoride, rubidium fluoride, cesium fluoride, etc.), alkali metal chlorides (e.g., lithium chloride, sodium chloride, potassium chloride, rubidium chloride, cesium chloride, etc.), alkali metal bromide (e.g., lithium bromide, sodium bromide, potassium bromide, rubidium chloride, cesium bromide, etc.), alkali metal iodide (e.g., lithium iodide, sodium iodide , potassium iodide, rubidium, and a cesium iodide, etc.) and the like. Preferable examples include the alkali metal chloride (e.g., lithium chloride, sodium chloride, potassium chloride, rubidium chloride, cesium chloride, etc.) and the like. Especially preferable examples include potassium chloride.

The amount of the alkali metal salt may be 1 to 10 mole equivalents, preferably 2 to 3 mole equivalents of Compound (VI).

Examples of the substituted or unsubstituted phenol include phenol, or phenol substituted with hydroxy or an electron withdrawing group (e.g., fluorine, chlorine, bromine, iodine, cyano, trifluoromethyl, nitro and the like).

Preferable examples include phenol, or phenol substituted with one or more substituent selected from the group consisting of fluorine; chlorine; bromine: iodine, hydroxy, cyano, trifluoromethyl and nitro. Ortho-chlorophenol, 2,4,6-trichlorophenol and the like are more preferable. Ortho-chlorophenol is particularly preferred.

A solvent is not limited as long as it efficiently proceeds in the above process. Examples of a solvent include 2-methyltetrahydrofuran, tetrahydrofuran, toluene, dioxane and the like. Preferable solvent includes 2-methyltetrahydrofuran, tetrahydrofuran, toluene and the like. Especially preferable solvent includes 2-methyltetrahydrofuran. A single solvent or mixed solvent can be used.

The temperature for such reaction is not limited, but usually can be about 0 to 100 °C and preferably about 50 to 80 °C.

Reaction time is not limited, but usually can be conducted for 0.5 to 12 hours and preferably 0.5 to 6 hour(s).

### Step 3

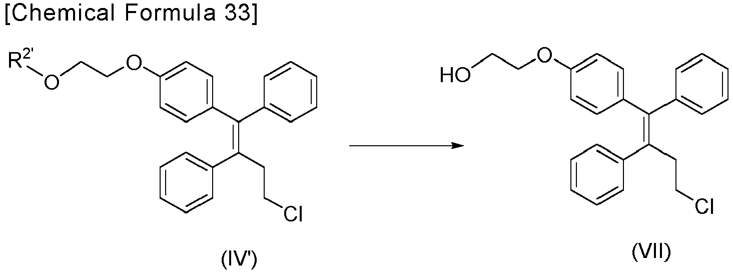

wherein R²' is a protecting group.

Compound (VII) can be obtained by hydrolyzing Compound (IV') in the presence of a base.

A base is not limited as long as it efficiently proceeds in the above process. Examples of base include sodium hydroxide, lithium hydroxide, potassium hydroxide, calcium hydroxide, cesium hydroxide and the like. Preferable examples include sodium hydroxide.

The amount of the base may be 1 to 5 mole equivalents, preferably 1 to 3 mole compared to equivalents of Compound (IV').

A solvent is not limited as long as it efficiently proceeds in the above process. One or more solvents selected from the group consisting of tetrahydrofuran, methanol, ethanol, water and the like can be used. Preferable solvent includes the solvent mixture of tetrahydrofuran and methanol. The solvent can be used as a two phase solvent with water or hydrous solvent, if necessary.

The temperature for such reaction is not limited, but usually can be about 0 to 50 °C and preferably at room temperature.

Reaction time is not limited, but usually can be conducted for 0.5 to 12 hours and preferably 1 to 5 hour(s).

### Step 2

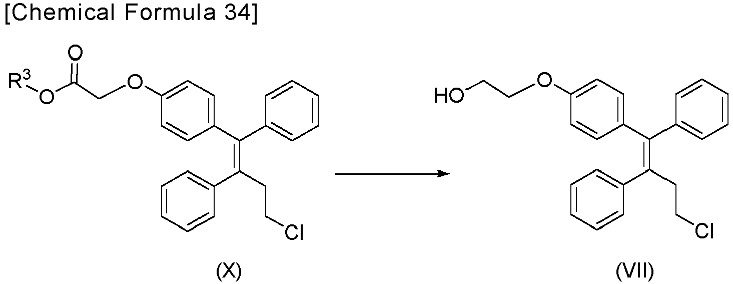

wherein R³ is a substituted or unsubstituted alkyl or a substituted or unsubstituted aromatic carbocyclyl.

Compound (VII) can be obtained by reducing Compound (X) with sodium borohydride.

The amount of the sodium borohydride can be 1 mol to 5 mol equivalent(s) to Compound (X).

A solvent is not limited as long as it efficiently proceeds in the above process. One or more solvents selected from the group consisting of methanol, ethanol, isopropanol, n-propanol, tert-butanol, n-butanol, 1,2-dimethoxyethane, N, N-dimethylformamide, N,N-dimethylacetoamide, N-methyl pyrrolidone, 1,3-dimethyl-2-imidazolidinone, toluene, cyclopentylmethylether, tetrahydrofuran, 2-methyl tetrahydrofuran, dimethylsulfoxide and the like can be used. The solvent can be used as a two phase solvent with water or hydrous solvent, if necessary. Preferable solvent includes polar solvent.

Examples of polar solvents include one or more solvents selected from the group consisting of methanol, ethanol, isopropanol, n-propanol, tert-butanol, n-butanol, 1,2-dimethoxyethane, N, N-dimethylformamide, N,N-dimethylacetoamide, N-methyl pyrrolidone, 1,3-dimethyl-2-imidazolidinone, cyclopentylmethylether, tetrahydrofuran, 2-methyl tetrahydrofuran, dimethylsulfoxide and the like can be used. Especially preferable solvent includes the solvent mixture of tetrahydrofuran and methanol.

The temperature for such reaction is not limited, but usually can be about 0 to 100 °C and preferably 0 °C to room temperature.

Reaction time is not limited, but usually can be conducted for 0.5 to 24 hours and preferably 1 to 10 hour(s).

### Example 1 (not according to the invention as claimed)

### The preparation of Ospemifene (Compound 2)

### Step 1

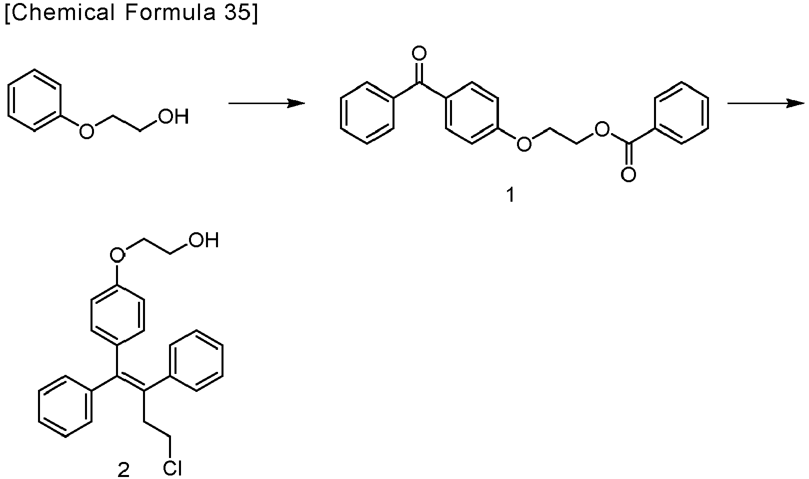

A solution of 2-phenoxyethanol (5.53 g, 40 mmol) in methylene chloride (50 mL) was added dropwise into a suspension of aluminum chloride (14.1 g, 100 mmol) in methylene chloride (50 mL) with ice-cooling under nitrogen atmosphere for 5 minutes. Then a solution of benzoyl chloride (13.3 g, 100 mmol) in methylene chloride (25 mL) was added dropwise for 5 minutes and the solution was stirred at room temperature for 2 hours. The reaction mixture was poured into ice (100 g), and concentrated hydrochloric acid (20 mL) was added and the mixture was stirred for 30 minutes at room temperature. Methylene chloride (20 mL) was added, and the mixture was extracted twice. The organic layer was washed with water (50 mL), and the combined organic layers were washed with 10% aqueous potassium carbonate solution (50 mL), then saturated aqueous sodium chloride solution (50 mL). The aqueous layer was extracted with methylene chloride (20 mL), and all organic layers were combined. The combined organic layers were dried over anhydrous magnesium sulfate. After filtration, the solvent was evaporated under reduced pressure to give pale pink oil (20 g). The oil was dissolved in ethyl acetate (20 mL), then hexane (40 mL) was added. Seed crystals A (about 3 mg) were added to the solution, and the solution was stirred for 5 minutes. Then hexane (40 mL) was added dropwise into the solution over 30 minutes. The precipitated solid was filtered, washed with the filtrate, then hexane - ethyl acetate (19:1) twice. The precipitated solid was dried to yield Compound 1 (12.3 g, yield: 88 .4%) as a white solid. The filtrate was concentrated under reduced pressure, and the obtained residue was purified by silica gel column chromatography (hexane-ethyl acetate). The obtained white solid was dissolved in ethyl acetate (3 mL), and then, hexane (27 mL) was added dropwise to the solution. The precipitated solid was filtered, then washed with hexane - ethyl acetate (19:1) twice to yield Compound 1 (1.11 g, yield: 8 .0%) as a white solid. Total yield: 13.4 g, (96.4%)

¹ H-NMR (CDCl₃) δ : 4.41 (t, J = 4.8 Hz, 2H), 4.71 (t, J = 4.8 Hz, 2H), 7.02 (d, J = 8.8 Hz, 2H), 7.42-7.50 (m, 4H), 7.57 (t, J = 7.5 Hz, 2H), 7.75 (d, J = 6.8 Hz, 2H), 7.84 (d, J = 8.8 Hz, 2H), 8.06 (d, J = 7.1 Hz, 2H).

### The preparation of seed crystals A

A solution of benzoyl chloride (16.9 g, 120 mmol) was added dropwise into a suspension of aluminum chloride (26.7 g, 200 mmol) in methylene chloride (100 mL) with ice-cooling under nitrogen atmosphere for 3 minutes, then 2-phenoxyethanol (5.53 g, 40 mmol) was added dropwise into the mixture for 3 minutes. The mixture was stirred at room temperature for 100 minutes. Then benzoyl chloride (5.62 g, 40 mmol) was added, and the mixture was stirred at room temperature for 40 minutes and at reflux for 130 minutes. Benzoyl chloride (5.62 g, 40 mmol) was added, and the mixture was stirred at reflux for 120 minutes. The mixture was poured into ice (200 g) and concentrated hydrochloric acid (20 mL) was added.

The mixture was stirred for 30 minutes at room temperature, and separated. The aqueous layer was again extracted with methylene chloride (50 mL). The organic layer was washed with the mixed solution of water (50 mL), saturated sodium chloride aqueous solution (50 mL) and 2 mol/L hydrochloric acid aqueous solution (100 mL). The organic layer was washed with saturated sodium chloride aqueous solution (50 mL) again. The organic layers were combined and dried over anhydrous magnesium sulfate. After filtration, the solvent was evaporated under reduced pressure to obtain yellow oil.

1/10 of the oil was purified by silica gel chromatography (hexane / ethyl acetate). The obtained oil was crystalized with hexane - ethyl acetate (17:3) (10 mL). The precipitated solid was filtered, and washed with hexane - ethyl acetate (17:3) (2 mL).

The precipitated solid was dried to yield seed crystals A of Compound 1 (1.24 g, yield: 8.95%) as a white solid.

9/10 of the oil was dissolved in ethyl acetate (27 mL), and hexane (64 mL) Then the seed crystals A, prepared above were added to give precipitate. Hexane (64 mL) was added. The precipitate was filtered, washed with the filtrate, then washed with hexane - ethyl acetate (17:3). The resulting solid was dried to yield Compound 1 (12.3 g, yield: 88 .4%) as a white solid. The filtrate was concentrated under reduced pressure to obtain yellow oil. The obtained oil was purified by silica gel column chromatography (hexane-ethyl acetate), and the solvent was evaporated under reduced pressure to give Compound 1 as a white solid (3.03 g, 21.9% yield).

### Step 2-1 The preparation of Compound 2 (Ospemifene)

Titanium tetrachloride (6.54 mL, 20 mmol) was added to the degassed mixture of toluene (26 mL) and 2-methyl tetrahydrofuran (21 mL) below 13 °C under N₂, and the mixture was stirred at 0 °C for 10 minutes. The obtained solution was Liquid A.

A solution of orthochlorophenol (7.71 g, 60 mmol) in 2-methyltetrahydrofuran (26 mL) was added to the suspension of Compound 1 (10.4 g, 30 mmol), zinc (7.85 g, 120 mmol), potassium chloride (8.95 g, 120 mmol) and 3-chloro-propiophenone (5.06 g, 30 mmol) in toluene (26 mL). The suspension was degassed three times.

Then Liquid A was added dropwise to the suspension below 11 °C, washed with 2-methyl tetrahydrofuran (5 mL). The suspension was degassed three times again. The suspension was heated from 0 to 70 °C for 45 minutes, and the suspension was stirred at 70 °C for 60 minutes. The suspension was cooled to room temperature, and toluene (25 mL) was added to the suspension. Water (100 mL) and concentrated hydrochloric acid (100 mL) was added with ice-cooling, and the mixture was stirred for 10 minutes at room temperature. The mixture was extracted with toluene (25 mL) twice, and the organic layer was washed with water (50 mL), 2 mol / L aqueous sodium hydroxide solution (100 mL), water (50 mL) and saturated aqueous sodium chloride solution (50 mL). The combined organic layers were dried over anhydrous magnesium sulfate, and the solvent was removed under reduced pressure to obtain yellow oily crude product (approximately 16 g).

The obtained oily crude product was dissolved in tetrahydrofuran-methanol (1:1, 80 mL), and then, 2 mol/L aqueous sodium hydroxide solution (30 mL) was added. The mixture was stirred at room temperature for 2 hours. Water - saturated aqueous sodium chloride (5:1, 90 ml) was added and the mixture was extracted with toluene (100 mL and 50 mL) twice. The organic layer was washed with water - saturated aqueous sodium chloride (5:1, 90 ml) and washed with saturated aqueous sodium chloride solution (25 mL). The combined organic layers were dried over anhydrous magnesium sulfate and filtered to obtain a solution (244 g) containing the crude product.

1/30 of the solution containing the obtained crude product was evaporated under reduced pressure, and the obtained residue was purified by silica gel column chromatography (hexanes-ethyl acetate) to obtain Compound 2 (249 mg, purity 99.5%, 66% in terms of yield, *Z E* = 4.2:1).

29/30 of the solution containing the crude product was evaporated under reduced pressure, and methanol (50 mL) was added to the residue. The mixture was evaporated under reduced pressure to obtain an oil (13.5 g). The oil was dissolved in methanol (57 mL). The mixture was headed to 50 °C, and water (14.2 mL) was added. The mixture was heated at 40 °C for 30 minutes. (When the temperature of solution was at 40 °C, and seed crystals A were added.) And then, the mixture was cooled at 15 °C for 60 minutes, stirred at the temperature for 60 minutes. The precipitate was filtered, washed with the filtrate, then methanol - water (3:1, 3 mL) twice and methanol - water (3:1, 2 mL). The obtained solid was dried with heating under reduced pressure to obtain Ospemifene as a white solid (8.4 g, *Z:E* = 11.6:1). The obtained Ospemifene was dissolved in methanol (44 mL) at 50 °C, and water (11 mL) was added dropwise to the mixture. The mixture was cooled to room temperature for 60 minutes and was standing for 14 hours. The mixture was stirred at 15 °C for 30 minutes, and filtered. The resulting solid was washed with the filtrate, then methanol - water (2:1, 4 mL) three times. The obtained solid was dried under heating under reduced pressure to obtain Ospemifene as a white solid (9.1 g, *Z:E* = 53.5:1). The obtained Ospemifene was dissolved in methanol (44 mL) at 58 °C, and then, water (11 mL) was added dropwise to the mixture. The mixture was cooled to room temperature for 15 °C for 30 minutes. The precipitate was filtered, washed with the filtrate, then washed with methanol - water (2:1, 5 mL) twice. The obtained solid was dried with heating under reduced pressure to obtain Ospemifene as a white solid (4.90 g, yield: 45%, *Z:E* = 341:1).

### Step 2-2 The preparation of Compound 2 (Ospemifene)

The 2-methyl tetrahydrofuran (15 mL) was degassed with nitrogen three times, and titanium tetrachloride (2.2 mL, 20 mmol) was added, and the mixture was stirred at 0 °C for 10 minutes. The obtained solution was Liquid A.

The suspension of Compound 1 (3.46 g, 10 mmol), zinc (2.62 g, 40 mmol), potassium chloride (2.98 g, 40 mmol), 3-chloro-propiophenone (1.69 g, 10 mmol) and orthochlorophenol (2.57 g, 20 mmol) in 2-methyl tetrahydrofuran (17.5 mL) was degassed with nitrogen three times. Then Liquid A was added dropwise to the suspension below 11 °C, washed with 2-methyl tetrahydrofuran (2.5 mL). The suspension was degassed with nitrogen three times again. The suspension was heated from 0 to 70 °C for 45 minutes, and stirred at 70 °C for 50 minutes. The suspension was cooled to room temperature and toluene (35 mL) was added to the suspension. Water (40 mL) and concentrated hydrochloric acid (40 mL) was added under ice-cooling, and the mixture was stirred for 30 minutes at room temperature.

The mixture was extracted with toluene (25 mL) twice, and the combined organic layers were washed with water (20 mL), 2 mol/L aqueous sodium hydroxide solution (40 mL), and saturated aqueous sodium chloride solution (20 mL).

The combined organic layers were dried over anhydrous magnesium sulfate, and the solvent was removed under reduced pressure to obtain yellow oily crude product.

The obtained crude product was dissolved in tetrahydrofuran-methanol (1:1, 30 mL), and then, 2 mol/L aqueous sodium hydroxide solution (12.5 mL) was added below 10 °C. The mixture was stirred at room temperature for 90 minutes. Water - saturated aqueous sodium chloride (5:1, 30 ml) was added and the mixture was extracted with toluene (35 mL and 25 mL) twice. The combined organic layers were washed with water - saturated aqueous sodium chloride (5:1, 30 ml) and washed with saturated aqueous sodium chloride solution (20 mL).

The combined organic layers were dried over anhydrous magnesium sulfate, filtered and the solvent was removed under reduced pressure.

The obtained residue was purified by silica gel column chromatography (hexane-ethyl acetate) to obtain Compound 2 (2.62 g, purity: 99.1%, yield: 69%, *Z:E* = 4.7:1).

### Step 2-3 The preparation of Compound 2 (Ospemifene)

In the above steps 2-2, Compound 2 was obtained using phenol (2.0 eq), instead of potassium chloride (4.0 eq) and orthochlorophenol (2.0 eq), (yield: 59%, *Z:E* = 4.6:1). Other than the above reagents, the same process as the above steps 2-2 was conducted.

### Step 2-4 The preparation of Compound 2 (Ospemifene)

In the above steps 2-2, Compound 2 was obtained using orthochlorophenol (2.0 eq), instead of potassium chloride (4.0 eq) and orthochlorophenol (2.0 eq), (yield: 67%, *Z:E* = 4.6:1). Other than the above reagents, the same process as the above steps 2-2 was conducted.

### Step 2-5 The preparation of Compound 2 (Ospemifene)

In the above steps 2-2, Compound 2 was obtained using the mixture of 2-methyltetrahydrofuran and toluene (2-methyltetrahydrofuran :toluene = 1:1), instead of 2-methyltetrahydrofuran, (yield: 68%, *Z:E* = 4.3:1). Other than the above solvent, the same process as the above steps 2-2 was conducted.

### Step 2-6 The preparation of Compound 2 (Ospemifene)

In the above steps 2-2, Compound 2 was obtained using 2, 4, 6-trichlorophenol (2.0 eq), instead of potassium chloride (4.0 eq) and orthochlorophenol (2.0 eq), (yield: 64%, *Z:E* = 4.8:1). Other than the above reagents, the same process as the above steps 2-2 was conducted.

### Step 2-7 The preparation of Compound 2 (Ospemifene)

In the above steps 2-2, Compound 2 was obtained using parachlorophenol (2.0 eq), instead of potassium chloride (4.0 eq) and orthochlorophenol (2.0 eq), (yield: 61%, *Z:E* = 4.8:1). Other than the above reagents, the same process as the above steps 2-2 was conducted.

### Example 2

### The preparation of Ospemifene 2

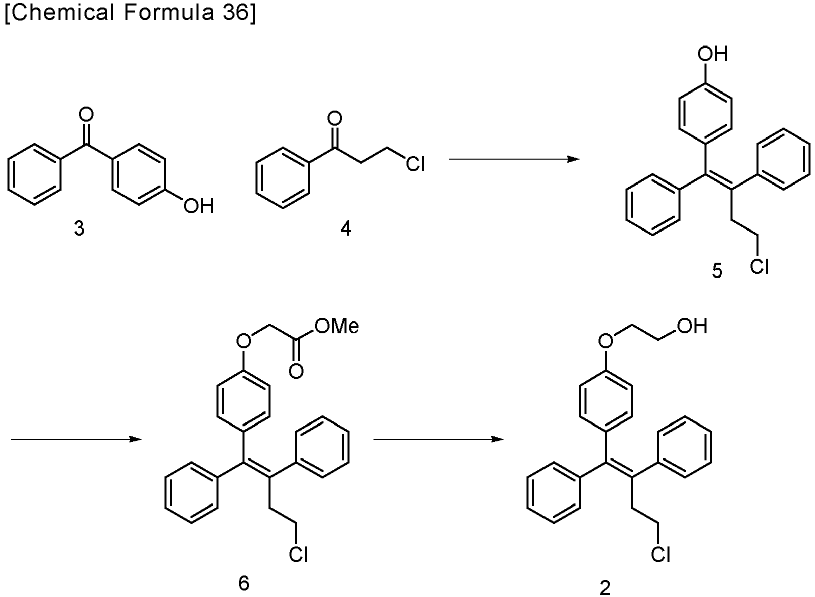

### Step 1-1 The preparation of Compound 5

Compound 3 (2.97 g, 15 mmol), Compound 4 (2.53 g, 15 mmol), zinc (3.73 g, 57 mmol) and potassium chloride (4.25 g, 57 mmol) were added to 2-methyltetrahydrofuran (15 mL). The mixture was degassed with nitrogen five times.

Titanium tetrachloride (3.14 mL, 28.5 mmol) was added for 30 minutes, then the mixture was stirred at room temperature for 20 minutes and at 50 °C for 2 hours. After cooling, concentrated hydrochloric acid (6.1 g) and water (16 mL) were added. After the insoluble materials were filtered, the filtrate was extracted with ethyl acetate. The organic layer was washed with water and saturated sodium chloride aqueous solution, and dried over anhydrous magnesium sulfate. The solvent was concentrated under reduced pressure, and a part (331 mg) of the obtained residue (7.18 g) was collected.

It was purified by silica gel column chromatography to obtain Compound 5 (160mg, *Z*:*E* = 5.7:1). Methanol - water was added to the rest, and then, the seeds obtained above were added to the solution to give a precipitate. The precipitate was filtered to obtain Compound 5 (4.98 g, Quantitative value: 69.2%, *Z*:*E* = 5.7:1) as a crude product.

### Step 1-2 The preparation of Compound 5

Compound 3 (14.87 g, 75.0 mmol), Compound 4 (12.65 g, 75 mmol), and zinc (18.64 g, 285 mmol) were added to 2-methyltetrahydrofuran (149 mL). The mixture was degassed with nitrogen five times. Titanium tetrachloride (26.48 g, 140 mmol) was added for about 2 hours, then the mixture was stirred for 3 hours.

After cooling, concentrated hydrochloric acid (30.34 g) and water (80.01 g) were added. After the insoluble materials were filtered, the filtrate was extracted with ethyl acetate. The organic layer was washed with water three times to obtain the organic layer (359.9 g). The organic layer was separated and several amount of solvent (119.69 g) was concentrated under reduced pressure. Methanol was added to the obtained residue. The solvent was concentrated under reduced pressure again. Methanol (33 mL) / water (13.5 mL) were added to the residue. The resulting precipitate was filtered to obtain Compound 5 (4.599 g, Quantitative value: 46.6%, *Z:E* = 19:1) as a crude product.

### Step 2-1 The preparation of Compound 6

The crude product of Compound 5 (4.98 g) was dissolved in N, N-dimethylformamide (25 mL), and 2- methyl bromoacetate (1.69 mL, 17.9 mmol) and potassium carbonate (3.08 g, 22.31 mmol) was added to the mixture. , The mixture was stirred at room temperature for 1 hour. Water was added to the reaction mixture, and the mixture was extracted with ethyl acetate. The organic layer was washed with water and saturated brine, dried over anhydrous sodium sulfate and filtered. The solvent was removed under reduced pressure to obtain Compound 6 as a crude product.

¹H-NMR (CDCl₃) δ: 2.92 (t, J = 7.4 Hz, 2H), 3.41 (t, J = 7.4 Hz, 2H), 3.75 (s, 3H), 4.50 (s, 2H), 6.55 (d, J = 8.6 Hz, 2H), 6.80 (d, J = 8.6 Hz, 2H).

### Step 2-2 The preparation of Compound 6

Compound 5 (200 mg, 0.597 mmol, Z: = 20:1) was dissolved in N, N-dimethylformamide (1 mL), 2- methyl bromoacetate (67.8 µL, 0.717 mmol) and potassium carbonate (99 mg, 0.717 mmol) was added to the mixture. The mixture was stirred at room temperature for 2 hour. Water was added to the reaction mixture, and the mixture was extracted with ethyl acetate. The organic layer was washed with water and saturated brine, dried over anhydrous sodium sulfate and filtered. The solvent was removed under reduced pressure. The residue was purified by silica gel column chromatography to obtain Compound 6 (243 mg, *Z*:*E* = 20:1).

¹ H-NMR (CDCl₃) δ: 2.92 (t, J = 7.4 Hz, 2H), 3.41 (t, J = 7.4 Hz, 2H), 3.75 (s, 3H), 4.50 (s, 2H), 6.55 (d, J = 8.6 Hz, 2H), 6.80 (d, J = 8.6 Hz, 2H), 7.08-7.43 (m, 10H).

### Measurement of X ray powder diffraction patterns

X ray powder diffractions of crystals obtained from each example were measured according to a measurement method of an X ray powder diffraction described as general test procedures in Japanese Pharmacopoeia. Measurement conditions are shown below.

### (Device)

MiniFlex600 manufactured by Rigaku.

### (Operations procedures)

Samples were measured under the following conditions.
Measurement method: Reflection method
Type of a light source: Cu tube
Used wavelengths: CuKα radiation
Tube currents: 15 mA
Tube voltage: 40 Kv
Sample plates: non-reflective sample plate, Silicon
Scan speed: 20.000° / minute
Scanning range: 4.000 - 40.0000°
Sampling width: 0.0200°

Crystal is characterized by the value of each diffraction angle or surface interval (dsinθ = nλ: n is an integer, d is the surface interval (unit: angstrom), θ is the diffraction angle (unit: refers to the degree)).

The results of the powder X-ray diffraction are shown in Table 1 and Figure 1. Main peak angle of Diffraction: 2θ = 18.0° ± 0.2°, 21.6° ± 0.2°, 22.1° ± 0.2°, 23.9° ± 0.2° and 25 .6° ± 0.2°

**Table 1**

| Angle 2-Theta ° | d value Ansgstrom | Intensity % |
|---|---|---|
| 8.8 | 10.04 | 24 |
| 18.0 | 4.94 | 31 |
| 21.6 | 4.10 | 83 |
| 22.1 | 4.03 | 39 |
| 22.2 | 4.01 | 30 |
| 22.7 | 3.92 | 27 |
| 22.9 | 3.87 | 21 |
| 23.9 | 3.72 | 100 |
| 24.5 | 3.63 | 28 |
| 25.5 | 3.49 | 30 |
| 25.6 | 3.48 | 62 |
| 26.4 | 3.37 | 29 |
| 27.2 | 3.27 | 21 |
| 27.4 | 3.25 | 29 |

### Step 3-1 The preparation of Compound 2 (Ospemifene)

Crude product of Compound 6 (6.05 g) was dissolved in tetrahydrofuran (30 mL) and methanol (30 mL). Sodium borohydride (1.13 g, 29.7 mmol) was added to the mixture at 0 °C, and the mixture was stirred at room temperature for 1 hour. 1 mol/L hydrochloric acid was added to the reaction mixture, and the mixture was extracted with ethyl acetate. The organic layer was washed with water and brine, dried with anhydrous sodium sulfate and filtered. The solvent was removed under reduced pressure, and a part of the obtained reside (8.06 g) was collected. It was purified by silica gel column chromatography to obtain Compound 2 (142 mg, *Z:E* = 7.8:1). Methanol - water was added to the rest, and the seeds obtained above was added to the solution to give a precipitate. The precipitate was filtered twice to obtain Compound 2 (2.42 g).

### Step 3-2 The preparation of Compound 2 (Ospemifene)

Compound 6 (100 mg, 0.246 mmol, Z:E = 20:1) was dissolved in mixture of tetrahydrofuran (1 mL) and methanol (1 mL). Sodium borohydride (18.6 mg, 0.492 mmol) was added to the mixture at 0 °C, and the mixture was stirred at room temperature for 3 hour. 1 mol/L hydrochloric acid was added to the reaction mixture, and the mixture was extracted with ethyl acetate. The organic layer was washed with water and brine, dried over anhydrous sodium sulfate and filtered. The solvent was removed under reduced pressure. The obtained reside was purified by silica gel column chromatography to obtain Compound 2 (93m g, quant, *Z*:*E* = 20:1).

### Step 3-3 The preparation of Compound 2 (Ospemifene)

Compound 6 (13.08 g, 32.14 mmol) was dissolved in tetrahydrofuran (65.4 mL). Sodium borohydride (2.43 g, 64.29 mmol) was added to the solution. Methanol was added to the mixture stirred at room temperature for 2 hours. The mixture was stirred for additional 30 minutes. 1 mol/L hydrochloric acid (78.5 mL) was added to the mixture, and then, the mixture was extracted with ethyl acetate twice. The combined organic layers were washed with water twice, and saturated aqueous sodium chloride solution, dried over anhydrous magnesium sulfate and filtered. The solvent was removed under reduced pressure to obtain Compound 2 (11.98 g, *Z:E* = 218:1) as a crude product. The crude product was crystalized from methanol - water, and the precipitated solid was filtered twice to obtain compound 2 (10.97 g, only Z).

### [Industrial Applicability]

The process of the present invention enables production Ospemifene and its intermediate with efficiency.

### [Brief Description of the Drawings]

Fig. 1 shows the pattern and the peak value of powder X-ray diffraction for crystal of compound obtained in Step 2-1 in Example 2. The vertical axis represents intensity, and the horizontal shows the diffraction angle [2θ, unit: °].

## Claims

1. A process for the preparation of a compound represented by formula (VII): 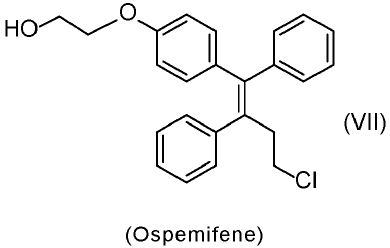 **characterized by** reacting a compound represented by Formula (X'): 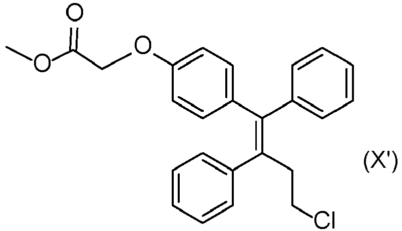 with sodium borohydride.

2. The process according to claim 1, wherein the amount of the sodium borohydride is from 1 mol to 5 mol equivalents to the compound of Formula (X').

3. The process according to claim 1 or claim 2, wherein the solvent is one or more solvents selected from the group consisting of methanol, ethanol, isopropanol, n-propanol, tert-butanol, n-butanol, 1,2-dimethoxyethane, N, N-dimethylformamide, N,N-dimethylacetoamide, N-methyl pyrrolidone, 1,3-dimethyl-2-imidazolidinone, toluene, cyclopentylmethylether, tetrahydrofuran, 2-methyl tetrahydrofuran, and dimethylsulfoxide.

4. The process according to claim 1 or claim 2, wherein the solvent is a polar solvent.

5. The process according to claim 4, wherein the solvent is one or more solvents selected from the group consisting of methanol, ethanol, isopropanol, n-propanol, tert-butanol, n-butanol, 1 ,2-dimethoxyethane, N, N-dimethylformamide, N,N-dimethylacetoamide, N-methyl pyrrolidone, 1,3-dimethyl-2-imidazolidinone, cyclopentylmethylether, tetrahydrofuran, 2-methyl tetrahydrofuran, and dimethylsulfoxide.

6. The process according to claim 4, wherein the solvent is a mixture of tetrahydrofuran and methanol.

## Patentansprüche

1. Verfahren zur Herstellung einer durch die Formel (VII) dargestellten Verbindung: 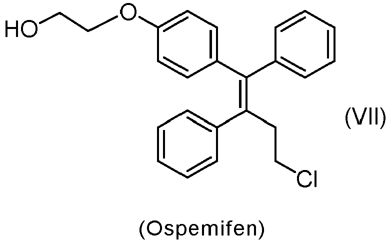 **dadurch gekennzeichnet, dass** eine Verbindung der Formel (X') mit Natriumborhydrid umgesetzt wird:

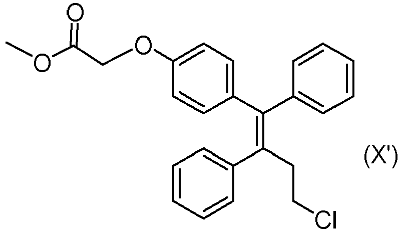

2. Verfahren nach Anspruch 1, wobei die Menge des Natriumborhydrids 1 bis 5 Molequivalente der Verbindung der Formel (X') beträgt.

3. Verfahren nach Anspruch 1 oder Anspruch 2, wobei das Lösungsmittel ein oder mehrere Lösungsmittel ausgewählt aus der Gruppe bestehend aus Methanol, Ethanol, Isopropanol, n-Propanol, tert-Butanol, n-Butanol, 1,2-Dimethoxyethan, N,N-Dimethylformamid, N,N-Dimethylacetoamid, N-Methylpyrrolidon, 1,3-Dimethyl-2-imidazolidinon, Toluol, Cyclopentylmethylether, Tetrahydrofuran, 2-Methyltetrahydrofuran, und Dimethylsulfoxid ist.

4. Verfahren nach Anspruch 1 oder Anspruch 2, wobei das Lösungsmittel ein polares Lösungsmittel ist.

5. Verfahren nach Anspruch 4, wobei das Lösungsmittel ein oder mehrere Lösungsmittel ausgewählt aus der Gruppe bestehend aus Methanol, Ethanol, Isopropanol, n-Propanol, tert-Butanol, n-Butanol, 1,2-Dimethoxyethan, N,N-Dimethylformamid, N,N-Dimethylacetoamid, N-Methylpyrrolidon, 1,3-Dimethyl-2-imidazolidinon, Cyclopentylmethylether, Tetrahydrofuran, 2-Methyltetrahydrofuran, und Dimethylsulfoxid ist.

6. Verfahren nach Anspruch 4, wobei das Lösungsmittel eine Mischung aus Tetrahydrofuran und Methanol ist.

## Revendications

1. Procédé de préparation d'un composé représenté par la formule (VII): 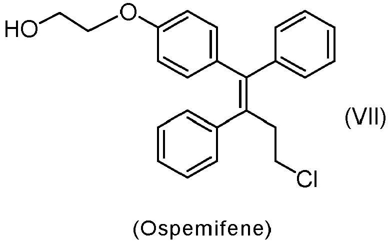 **caractérisé par** la réaction d'un composé représenté par la formule (X'): 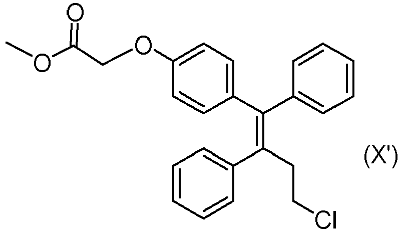 avec du borohydrure de sodium.

2. Procédé selon la revendication 1, dans lequel la quantité de borohydrure de sodium est de 1 mol à 5 mol équivalents au composé de formule (X').

3. Procédé selon la revendication 1 ou la revendication 2, dans lequel le solvant est un ou plusieurs solvants choisis dans le groupe constitué par le méthanol, l'éthanol, l'isopropanol, le n-propanol, le tert-butanol, le n-butanol, le 1,2-diméthoxyéthane, N, N-diméthylformamide, N,N-diméthylacétoamide, N-méthyl pyrrolidone, 1,3-diméthyl-2-imidazolidinone, toluène, cyclopentylméthyléther, tétrahydrofurane, 2-méthyl tétrahydrofurane, et diméthylsulfoxyde.

4. Procédé selon la revendication 1 ou la revendication 2, dans lequel le solvant est un solvant polaire.

5. Procédé selon la revendication 4, dans lequel le solvant est un ou plusieurs solvants choisis dans le groupe constitué par le méthanol, l'éthanol, l'isopropanol, le n-propanol, le tert-butanol, le n-butanol, le 1,2-diméthoxyéthane, N, N-diméthylformamide, N,N-diméthylacétoamide, N-méthylpyrrolidone, 1,3-diméthyl-2-imidazolidinone, cyclopentylméthyléther, tétrahydrofurane, 2-méthyltétrahydrofurane, et diméthylsulfoxyde.

6. Procédé selon la revendication 4, dans lequel le solvant est un mélange de tétrahydrofurane et de méthanol.
